# EUROPÄISCHE PATENTANMELDUNG

(11) **EP 1 686 502 A1**
(43) Veröffentlichungstag der Anmeldung: **02.08.2006**
(21) Anmeldenummer: 06001631.8
(22) Anmeldetag: 26.01.2006
(51) Int. Cl.: G06F 19/00

(54) **Überwachungssystem zur Erfassung von physiologischen Parametern einer Person**

(30) Priorität: 31.01.2005 DE 102005004444
(71) Anmelder: Actimon GmbH & Co. KG, 69190 Walldorf (DE)
(72) Erfinder: Wegertseder, Dominik, 85540 Haar (DE); Schweizer, Thomas, 85560 Ebersberg (DE)
(74) Vertreter: Müller-Boré & Partner Patentanwälte

(57) **Zusammenfassung**

Überwachungssystem zur Erfassung von physiologischen Parametern einer Person, umfassend
- zumindest eine an der Person angeordnete, Datenerfassungseinrichtung, welche ausgelegt ist, physiologischen Parameter zu erfassen und entsprechende Parameterdaten zu erzeugen;
- zumindest eine mobile Verarbeitungseinrichtung, welche mit der Datenerfassungseinrichtung in Signalverbindung steht und zum Empfang zumindest eines Teils der Parameterdaten ausgelegt ist;
- zumindest eine Eingabeeinrichtung, welche mit der Datenerfassungseinrichtung und/oder der Verarbeitungseinrichtung in Signalverbindung steht und über welche mit den erfassten physiologische Parametern in Zusammenhang stehende Zusatzinformation eingebbar sind;
   und
- zumindest eine ortsferne Auswerteeinrichtung zum Speichern zumindest eines Teils der der Verarbeitungseinrichtung übermittelten Daten.

## Beschreibung

Die vorliegende Erfindung betrifft ein Überwachungssystem zur Erfassung von physiologischen Parametern einer Person, ein Überwachungsverfahren zur Erfassung von physiologischen Parametern einer Person sowie ein entsprechendes Computerprogrammprodukt.

Überwachungssysteme zur Erfassung von physiologischen Parametern einer Person sind aus dem Stand der Technik bekannt. Diese sind jedoch zumeist einfach und rudimentär ausgebildet. Insbesondere hat sich zur Überwachung von physiologischen Parametern ein Tagebuch durchgesetzt, durch welches Zeitpunkt und Menge von eingenommenen Medikamenten sowie gegebenenfalls weitere durch den Patienten oder Arzt- bzw. Pflegepersonal einzutragende Messdaten erfasst werden.

Es ist Aufgabe der vorliegenden Erfindung, ein Überwachungssystem zur Erfassung von physiologischen Parametern einer Person sowie ein Überwachungsverfahren zur Erfassung von physiologischen Parametern einer Person vorzuschlagen, mittels welchen der körperliche Zustand einer Person in Langzeit zuverlässig erfasst werden kann.

Diese Aufgabe wird durch ein Überwachungssystem zur Erfassung von physiologischen Parametern einer Person gemäß Anspruch 1 , ein Überwachungsverfahren zur Erfassung von physiologischen Parametern einer Person gemäß Anspruch 11 sowie eine Computerprogrammprodukt gemäß Anspruch 13 gelöst. Bevorzugte Ausführungsformen ergeben sich aus den abhängigen Unteransprüchen.

Erfindungsgemäß ist ein Überwachungssystem zur Erfassung von physiologischen Parametern einer Person vorgesehen, umfassend
- zumindest eine an der Person angeordnete Datenerfassungseinrichtung, welche ausgelegt ist, einen physiologischen Parameter zu erfassen und entsprechende Parameterdaten zu erzeugen;
- zumindest eine mobile Verarbeitungseinrichtung, welche mit der Datenerfassungseinrichtung in Signalverbindung steht und zum Empfang zumindest eines Teils der Parameterdaten ausgelegt ist;
- zumindest eine Eingabeeinrichtung, welche mit der Datenerfassungseinrichtung und/oder der Verarbeitungseinrichtung in Signalverbindung steht und in welche mit dem zumindest einen erfassten physiologischen Parameter in Zusammenhang stehende Zusatzinformationen eingebbar sind und
- zumindest eine ortsferne Auswerteeinrichtung zum Speichern zumindest eines Teils der der Verarbeitungseinrichtung übermittelten Daten.
   Die Datenerfassungseinrichtung ist Vorteilhafterweise ausgebildet, verschiedenste und beliebig vorbestimmbare physiologische Parameter der Person zu erfassen. Hierzu können insbesondere der Blutdruck, der Puls, die Temperatur, die Atmungsfrequenz, das EKG (d.h. die Spannungsschwankung bzw. Stromspannungsschwankung im Körper bzw. im/am Herz) und der Sauerstoffsättigungsgehalt des Blut zählen. Die Datenerfassungseinrichtung ist vorzugsweise mobil ausgebildet und am Objekt angeordnet bzw. anordenbar. Vorzugsweise kann diese auch in unmittelbarer räumlicher Nähe zu diesem angeordnet sein, wobei unter "unmittelbarer räumlicher Nähe" vorzugsweise ein Bereich bis zu ca. 100 m, besonders bevorzugterweise ca. 20 m, weiterhin besonders bevorzugterweise bis ca. 5 m und ganz besonders bevorzugterweise weniger als ca. 1 m zu verstehen ist. Die Datenerfassungseinrichtung ist ausgelegt, aus den physiologischen Parametern so genannte Parameterdaten zu erzeugen. Die Parameterdaten können somit vorzugsweise ein aus den erfassten physiologischen Parametern abgewandeltes analoges oder digitales Datensignal darstellen.
   Die Verarbeitungseinrichtung ist Vorteilhafterweise mobil ausgebildet, so dass diese - dem Objekt zugeordnet - mit dem Objekt bewegbar ist. Insofern ist unter "mobil" vorzugsweise ein Zustand zu verstehen, in welchem die Verarbeitungseinrichtung sowohl hinsichtlich ihrer räumlichen Abmessungen als auch hinsichtlich ihres Gewichts durch eine Person bewegbar ist. Vorzugsweise ist die mobile Verarbeitungseinrichtung leichter als ca. 2,5 kg und besonders bevorzugterweise leichter als ca. 500gr und meist bevorzugterweise leichter als ca. 300gr. Die räumlichen Abmessungen bzw. die räumliche Ausdehnung der Verarbeitungseinrichtung ist Vorteilhafterweise geringer als ca. 0,1 m² und besonders bevorzugterweise geringer als ca. 0,001 m². In einer besonders bevorzugten Ausführungsform ist die mobile Verarbeitungseinrichtung als Mobiltelefon oder PDA (personal digital assistant) ausgebildet. Die an der Person angeordnete Datenerfassungseinrichtung ist Vorteilhafterweise kompakt ausgebildet und eigenständig an der Person angeordnet, d.h. unabhängig von der Position der weiteren Elemente des Überwachungssystems positionierbar. Die Datenerfassungseinrichtung und die Verarbeitungseinrichtung können somit separat ausgebildet sein; es ist jedoch ebenfalls möglich, die Datenerfassungseinrichtung und die Verarbeitungseinrichtung integral bzw. einstückig bzw. als eine Einheit auszubilden. Somit ist es Vorteilhafterweise möglich, die Datenerfassungseinrichtung so anzuordnen, dass die Person möglichst geringfügig beeinflusst wird. Die mobile Verarbeitungseinrichtung kann Vorteilhafterweise einerseits an der Person selbst oder dieser benachbart angeordnet sein. Die Signalverbindung zwischen der Verarbeitungseinrichtung und der Datenerfassungseinrichtung ermöglicht es, eine Vielzahl von verschiedenen Daten zu übertragen, wobei diese Daten bzw. Parameterdaten einerseits in Form von Einzeldaten oder ganzen Datensätzen vorgesehen werden können. Als Einzeldaten können beispielsweise der Blutdruck oder Puls eines bestimmten Zeitpunkts übertragen werden, wohingegen als Datensatz beispielsweise der Verlauf des Blutdrucks oder Pulses der Person über einen vorbestimmten bzw. vorbestimmbaren Zeitraum übertragen werden kann.
   Über die Eingabeeinrichtung sind Vorteilhafterweise mit den erfassten physiologischen Parametern in Zusammenhang stehende Zusatzinformationen eingebbar. Derartige Zusatzinformationen können beispielsweise der Gemütszustand oder die Einnahmezeiten und/oder -mengen von Medikamenten sein. Die Eingabeeinrichtung ist somit vorteilhafterweise passiv ausgebildet, d. h. ausgelegt, von aktiver dritter Stelle Zusatzinformationen zu erhalten. Hierbei ist aktiv dahingehend zu verstehen, dass eine aktive Handlung, d.h. eingeben von Daten etc., der Person erforderlich ist. Die Datenerfassungseinrichtung hingegen ist Vorteilhafterweise aktiv ausgebildet, d. h. ausgelegt, die physiologischen Parameter eigenständig zu erfassen, so dass die Person bei der Erfassung der physiologischen Parameter im Wesentlichen passiv ist. Die mittels der Eingabeeinrichtung eingegebenen Zusatzinformationen können über eine Signalverbindung Vorteilhafterweise der Datenerfassungseinrichtung und/oder der Verarbeitungseinrichtung übermittelt werden.
   In einer besonders bevorzugten Ausführungsform weist die Verarbeitungseinrichtung eine Möglichkeit zur Eingabe von Zusatzinformationen auf.
   Die Auswerteeinrichtung ist zur Person Vorteilhafterweise Ortsfern angeordnet. Ortsfern bedeutet in diesem Zusammenhang vorzugsweise, dass hiervon ein Bereich von mindestens ca. 20 m, bevorzugterweise mindestens ca. 50 m und besonders bevorzugterweise mindestens ca. 1 km umfasst wird. Die Auswerteeinrichtung kann einerseits in obigem Sinne mobil (beispielsweise in Form eines Laptops) oder alternativ auch immobil ausgebildet sein, beispielsweise in einem extern angeordneten Rechenzentrum. Die Auswerteeinrichtung ist ausgelegt, zumindest einen Teil der der Datenverarbeitungseinrichtung übermittelten Daten zu speichern. Somit dient die Auswerteeinrichtung Vorteilhafterweise der Protokollierung der Daten, welche durch die Datenerfassungseinrichtung und/oder die Eingabeeinrichtung erfasst bzw. übermittelt wurden.
   Bevorzugterweise weist die Verarbeitungseinrichtung und/oder die Datenerfassungseinrichtung eine Feedbackeinrichtung auf, welche eine Ausgabeeinrichtung zum Vermitteln von Feedbackinformationen an die Person umfasst. Die Feedbackeinrichtung ist Vorteilhafterweise ausgelegt, Daten in Zusammenhang mit den an die ortsferne Auswerteeinrichtung gesendeten Daten zu empfangen. Diese Daten können Vorteilhafterweise der Datenerfassungseinrichtung zugeführt werden, insbesondere um diese zu steuern bzw. den Messvorgang zu regeln. Darüber hinaus ist es Vorteilhafterweise möglich, Feedbackinformationen über eine Ausgabeeinrichtung an die Person auszugeben. Diese Feedbackinformationen können insbesondere in Form von Anweisungen an den Patienten gestaltet sein, beispielsweise können Einnahmezyklen von Medikamenten ausgegeben werden. Die Feedbackeinrichtung muss jedoch nicht notwendigerweise Informationen von der ortsfernen Auswerteeinrichtung erhalten, sondern kann auch eigenständig Informationen an den Benutzer bzw. die Person ausgeben. Insbesondere kann die Feedbackeinrichtung ausgelegt sein, in der Datenerfassungseinrichtung bzw. der Verarbeitungseinrichtung Informationen zu gewinnen und in Abhängigkeit von diesen Informationen entsprechend Feedbackinformationen an die Person auszugeben. Somit würden Feedbackeinrichtung und Datenerfassungseinrichtung bzw. Verarbeitungseinrichtung ein in sich autarkes System darstellen.
   Bevorzugterweise sind die Verarbeitungseinrichtung und/oder die Datenerfassungseinrichtung ausgebildet, Auswertedaten von der Auswerteeinrichtung zu erhalten. Die Auswertedaten werden Vorteilhafterweise von der Ausgabeeinrichtung der Person angezeigt. Die Auswertedaten können beispielsweise vitale Parameter, d. h. die erfassten physiologischen Parameter bzw. eine zeitliche Abfolge oder ein Trend hiervon sein. Ebenfalls können als Auswertedaten Empfehlungen oder Erinnerungen ausgegeben werden, dass eine Behandlung, eine Messung oder Eingabe erfolgen soll.
   Vorteilhafterweise können die Auswertedaten auch hiermit in Zusammenhang stehende (Rohdaten) sein, welche in der Verarbeitungseinrichtung bzw. Datenerfassungseinrichtung weiter verarbeitet werden können.
   Vorzugsweise umfasst die Feedbackeinrichtung Erinnerungsmittel, welche ausgelegt sind, über die Ausgabeeinrichtung ein Erinnerungssignal auszugeben, welches die Person auffordert, eine vorzugsweise vorbestimmbare Aktion durchzuführen. Die Erinnerungsmittel können Vorteilhafterweise mit einem Timer bzw. einem Zeitsignalgeber versehen sein, um in zeitlicher Abhängigkeit ein Erinnerungssignal auszugeben. Die vorzugsweise vorbestimmbare Aktion kann in einer Dateneingabe über die Eingabeeinrichtung erfolgen. Es können somit insbesondere Daten bezüglich physiologischer Parameter, wie z.B. Gewicht, Gemütszustand, Schmerzen und Schlafverhalten eingegeben werden, sowie Daten bezüglich der Medikamenteneinnahme (Zeit, Menge) Darüber hinaus kann in der vorbestimmbaren Aktion auch ein aktives Handeln liegen, beispielsweise eine Aufforderung, eine gymnastische Übung durchzuführen.
   Bevorzugterweise sind die Erinnerungsmittel ausgebildet, das Erinnerungssignal nach einem vorbestimmten bzw. vorbestimmbaren Algorithmus auszugeben. Alternativ oder zusätzlich können die Erinnerungsmittel vorzugsweise ausgebildet sein, dass Erinnerungssignal in Abhängigkeit von den Parameterdaten, den Zusatzinformation und/oder den Auswertedaten auszugeben. Ein vorbestimmbarer Algorithmus kann dahingehend gegeben sein, dass das Erinnerungssignal in zeitlicher Abhängigkeit ausgegeben wird, z.B. täglich um 8 Uhr morgens zur Medikamenteneinnahme. Der vorbestimmbare Algorithmus kann jedoch auch adaptiv oder selbstlernend ausgebildet sein, nämlich in Abhängigkeit von den durch die Datenerfassungseinrichtung erfassenden physiologischen Parametern, beispielsweise eine Mengenanpassung der einzunehmenden Medikamente in Abhängigkeit von dem eingegebenen Gemütszustand oder erfassten Blutdruck. Vorzugsweise ist es jedoch auch möglich, das Erinnerungssignal von einer externen Stelle initiiert auszugeben, beispielsweise durch eine Datenbank in der Auswerteeinrichtung oder extern durch einen Arzt, welcher über die Auswertedaten die Ausgabe eines Erinnerungssignals bewirkt. Das Erinnerungssignal kann auch Vorteilhafterweise in Abhängigkeit von den Parameterdaten oder den eingegebenen Zusatzinformationen erfolgen, beispielsweise über einen Algorithmus, welcher abhängig von den Parameterdaten und/oder Zusatzinformation ein Erinnerungssignal ausgibt.
   In einer bevorzugten Ausführungsform ist die Eingabeeinrichtung als Ausgabevorrichtung für Medikamente, vorzugsweise als halb- oder vollautomatischer Medikamentenspender ausgebildet, welche ausgelegt ist, zumindest einen Teil der Zusatzinformationen an die Datenerfassungseinrichtung und/oder Verarbeitungseinrichtung zu übermitteln. Als halbautomatischer Medikamentenspender kann somit eine Vorrichtung vorgesehen werden, welche Eingabemittel zur zusätzlichen Eingabe des Medikamentenausgabezeitpunkts oder einer mit dieser in Zusammenhang stehenden Zusatzinformation nach oder vor der Ausgabe eines Medikaments durch die Person aufweist und ausgebildet ist, die Zusatzinformation an die Datenerfassungseinrichtung und/oder Verarbeitungseinrichtung zu übermitteln. Ein vollautomatischer Medikamentenspender hingegen ist Vorteilhafterweise ausgebildet, vor, nach oder während der Medikamentenausgabe eigenständig bzw. autonom bzw. direkt die Zusatzinformationen an die Datenerfassungseinrichtung und/oder Verarbeitungseinrichtung eigenständig zu übermitteln. Die Zusatzinformationen können hierbei insbesondere der Zeitpunkt der Medikamentenausgabe sowie die ausgegebene Menge sein. Darüber hinaus ist es möglich, Zusatzinformationen an die Datenerfassungseinrichtung und/oder Verarbeitungseinrichtung zu übermitteln, welche der Person einen Zeitpunkt vorgeben, wann das Medikament zu nehmen ist bzw. eine vorbestimmte bzw. vorbestimmbare Aktion durchgeführt werden muss. Es kann somit ein Überwachungs- und Medikamentenausgabesystem zur Erfassung von physiologischen Parametern einer Person in Abhängigkeit deren Medikamenteneinnahme vorgesehen, umfassend
- zumindest eine an der Person angeordnete Datenerfassungseinrichtung, welche ausgelegt ist, einen physiologischen Parameter zu erfassen und entsprechende Parameterdaten zu erzeugen;
- zumindest eine mobile Verarbeitungseinrichtung, welche mit der Datenerfassungseinrichtung in Signalverbindung steht und zum Empfang zumindest eines Teils der Parameterdaten ausgelegt ist;
- zumindest einen Ausgabevorrichtung für Medikamente, vorzugsweise als halb- oder vollautomatischer Medikamentenspender ausgebildet, welche ausgelegt ist, Zusatzinformationen an die Datenerfassungseinrichtung und/oder Verarbeitungseinrichtung zu übermitteln, wobei die Zusatzinformationen derart festgelegt sind, daß diese mit dem zumindest einen erfaßten physiologischen Parameter in Zusammenhang stehen, und
- zumindest eine ortsferne Auswerteeinrichtung zum Speichern zumindest eines Teils der der Verarbeitungseinrichtung übermittelten Daten.

In einer weiteren vorteilhaften Ausführungsform ist die Ausgabeeinrichtung ausgebildet, als Feedbackinformation zumindest einen Teil der Parameterdaten und/oder zumindest einen Teil der Zusatzinformationen und/oder zumindest einen Teil der Auswertedaten auszugeben. Dies ist insbesondere vorteilhaft, da es nicht immer wünschenswert ist, der Person sämtliche der erfassten Daten auszugeben, sondern lediglich einen vorbestimmbaren Teil hiervon. So ist es beispielsweise möglich, von den Parameterdaten über die Ausgabeeinrichtung die Herzfrequenz sowie den Blutdruck anzuzeigen, jedoch weitere, sehr viel kompliziertere Daten direkt (d. h. ohne Ausgabe über die Ausgabeeinrichtung) an die Auswerteeinrichtung zu übermitteln.

In einer bevorzugten Ausführungsform ist die Ausgabeeinrichtung eine optische und/oder akustische Ausgabeeinrichtung. Weiterhin ist die Eingabeeinrichtung vorzugsweise eine manuelle, insbesondere in Form einer Tastatur, und/oder akustische Ausgabeeinrichtung. Die Ausgabeeinrichtung kann Vorteilhafterweise mit der Eingabeeinrichtung integral ausgebildet sein, beispielsweise in Form eines so genannten Touch Screens. Ebenfalls ist es möglich, die Ausgabeeinrichtung und/oder Eingabeeinrichtung lediglich akustisch zu steuern, beispielsweise über ein Mikrophon und einen Lautsprecher. Hierbei ist es besonders vorteilhaft, ein Spracherkennungssystem in der Eingabeeinrichtung vorzusehen.

Bevorzugterweise umfassen die Auswerteeinrichtung, die Verarbeitungseinrichtung und/oder die Datenerfassungseinrichtung eine Speichereinrichtung. Mittels der Speichereinrichtung ist es Vorteilhafterweise möglich, die Parameterdaten und/oder die Zusatzinformationen zu speichern, vorzugsweise über einen längeren Zeitraum, um eine Protokollierung dieser Daten zu ermöglichen. Als Speichereinrichtung können insbesondere sämtliche flüchtigen und nicht-flüchtigen elektronischen Halbleiterspeicher, insbesondere DRAMs und Flash-Speicher verwendet werden.

Vorteilhafterweise weisen die Verarbeitungseinrichtung, die Datenerfassungseinrichtung und/oder die Auswerteeinrichtung Funkübertragungsmittel auf. Diese können bevorzugterweise so ausgebildet sein, dass die Signalverbindung zwischen Datenerfassungseinrichtung und Verarbeitungseinrichtung mittels Bluetooth, WLAN oder Infrarotübertragung erfolgt. Die Übertragung der Daten zwischen Verarbeitungseinrichtung und ortsferner Auswerteeinrichtung kann vorzugsweise über GSM, GPRS oder WLAN erfolgen. In einer besonders bevorzugten Ausführungsform können die Daten von der Verarbeitungseinrichtung über WLAN an eine Übertragungseinheit gesendet werden, welche entsprechend über eine vorzugsweise drahtgebundene Verbindung (z.B. Internet) mit der Auswerteeinrichtung in Verbindung steht.

Vorteilhafterweise umfasst die Datenerfassungseinrichtung einen Sensor. Der Sensor ist insbesondere Vorteilhafterweise ausgelegt, eine oder mehrere der Messparameter wie Blutdruck, Puls, Sauerstoffsättigungsgehalt des Bluts, Temperatur, Atmungsfrequenz, EKG und Lage der Person zu erfassen. Zur Erfassung der Lage einer Person bzw. des Zustands dieser können Vorteilhafterweise Sensoren verwendet werden, welche die Beschleunigung und/oder die Drehrate, vorzugsweise jeweils in drei Achsen bzw. Achsrichtungen, messen. Die drei Achsrichtungen sind Vorteilhafterweise so angeordnet, dass sie den dreidimensionalen Raum aufspannen. Vorzugsweise stehen die Achsrichtungen senkrecht zueinander. Besonders bevorzugterweise eignen sich hierfür so genannte mikromechanische "LOW-g"-Halbleiter-Beschleunigungssensoren, welche eine Auflösung von wenigen Milli-g bzw. weniger als einem Grad pro Sekunde aufweisen. Es ist somit Vorteilhafterweise möglich, die Rotationsgeschwindigkeit und/oder - beschleunigung um eine oder mehrere Achsen zu messen. Darüber hinaus ist es möglich, die Position bzw. Lage des Körpers der Person zu ermitteln.

Vorteilhafterweise ist die Signalverbindung zumindest zwischen Verarbeitungseinrichtung und Auswerteeinrichtung als gesicherte Verbindung ausgebildet. Durch die gesicherte Verbindung kann Vorteilhafterweise die Authentizität der übermittelten Daten gewährleistet werden, so dass der Ursprung der Daten ermittelt bzw. geprüft bzw. verifiziert werden kann. Zur Sicherung der Authentizität ist es möglich, die codierten Daten mit einer Signatur zu versehen, beispielsweise mittels eines so genannten private-public-key-Verfahrens. Darüber hinaus kann die Verbindung gesichert sein, in dem die Daten für Dritte bzw. Außenstehende unlesbar sind, beispielsweise durch eine Verschlüsselung. Als Verschlüsselung kann hier insbesondere ein so genanntes public-private-key-Verfahren verwendet werden, so dass es lediglich dem Empfänger der Daten möglich ist, die Daten wieder zu entschlüsseln. Ferner kann zur Erkennbarkeit einer Manipulation die Daten mit einer Check bzw. Prüfsumme versehen werden. Zur Sicherung der Verbindung kann eine der oben genannten Sicherungen vorgesehen werden. In einer bevorzugten Ausführungsform sind jedoch sämtliche der drei oben genannten Sicherungsmechanismen durch die gesicherte Verbindung vorgesehen. Somit wird eine zuverlässige gesicherte Verbindung zumindest zwischen der Verarbeitungseinrichtung und Auswerteeinrichtung gewährleistet.

Vorteilhafterweise wird die gesicherte Verbindung über in der Verarbeitungseinrichtung, der Auswerteeinrichtung und/oder der Datenerfassungseinrichtung vorzugsweise modular angeordnete Kodierungsmittel bereitgestellt. Insbesondere können die Kodierungsmittel Vorteilhafterweise die Daten mittels einer Signatur versehen. Die Signatur kann beispielsweise über eine der Datenerfassungseinrichtung eindeutig zuordenbare elektronische Seriennummer erfolgen oder mittels eines beliebigen anderen Zertifikats. Somit wäre es beispielsweise möglich, die Eichdaten der Datenerfassungseinrichtung Vorteilhafterweise zum Bestandteil der Zertifikats zu machen, so dass das Eichdatum bzw. die Eichfrist die Laufzeit des Zertifikats bestimmt. Darüber hinaus wäre es ebenfalls möglich, biometrische Daten physiologischen Parametern (wie z.B. Herzschlag, Fingerabdrucksmuster oder Muster der Iris) zu nutzen, um eine Signatur der Daten zu ermöglichen. Somit ist es Vorteilhafterweise möglich, die Signatur durch Mittel vorzusehen, welche personen-spezifisch ausgebildet sind. Infolgedessen wird eine direkte und eindeutige Zuordnung zu der Person Vorteilhafterweise möglich. Durch eine vorzugsweise modulare Anordnung der Kodierungsmittel wird ein Vorteilhafterweise sehr variables System geschaffen, da verschiedenste Kodierungsmittel eingesetzt werden können. Vorzugsweise erfolgt die modulare Anordnung durch ein Stecksystem (slot), in welches entsprechende Kodierungsmittel eingeführt werden können. Besonders geeignet sind hierbei insbesondere MMC-cards, compact flash, memory sticks, SD-cards und SIM-Karten.

Weiterhin erfindungsgemäß ist ein Überwachungsverfahren zur Erfassung von physiologischen Parametern einer Person vorgesehen, umfassend die Schritte:
- Erfassen von physiologischen Parametern einer Person durch zumindest eine an dieser angeordneten Datenerfassungseinrichtung
- Erzeugen entsprechender Parameterdaten in Abhängigkeit der erfaßten physiologischen Parameter
- Empfangen zumindest eines Teils der Parameterdaten durch eine mobile Verarbeitungseinrichtung;
- Speichern zumindest eines Teils der der Verarbeitungseinrichtung übermittelten Parameterdaten in einer ortsfernen Auswerteeinrichtung;
- in Signalverbindung bringen zumindest einer Eingabeeinrichtung mit der Datenerfassungseinrichtung und/oder der Verarbeitungseinrichtung; und
- Eingeben von mit dem zumindest einen erfaßten physiologischen Parameter in Zusammenhang stehenden Zusatzinformationen

Es wird somit Vorteilhafterweise ein Überwachungsverfahren geschaffen, in welchem Daten über zwei verschiedene Einrichtungen dem Überwachungssystem zugeführt werden. Einerseits werden durch die Datenerfassungseinrichtung die physiologischen Parameter erfasst, ohne eine Aktivität der Person zwingend vorauszusetzen. Andererseits ist Vorteilhafterweise eine Aktivität der Person oder eines Dritten beim Eingeben von Zusatzinformationen erforderlich. Durch Speichern der Informationen und Übermitteln der Daten an die ortsferne Auswerteeinrichtung wird vorteilhafterweise ein Überwachungsverfahren geschaffen, welches eine zuverlässige Überwachung der Person und Protokollierung der Daten ermöglicht.

Bevorzugterweise umfasst das Überwachungsverfahren den Schritt:
- Ausgeben eines Erinnerungssignals durch Erinnerungsmittel einer Feedbackeinrichtung der Verarbeitungseinrichtung und/oder Datenerfassungseinrichtung über eine Ausgabeeinrichtung.

Ebenfalls bevorzugterweise kann ein Überwachungs- und Medikamentenausgabeverfahren zur Erfassung von physiologischen Parametern einer Person in Abhängigkeit deren Medikamenteneinnahme vorgesehen sein, umfassend die Schritte:
- Erfassen von physiologischen Parametern einer Person durch zumindest eine an dieser angeordneten Datenerfassungseinrichtung
- Erzeugen entsprechender Parameterdaten in Abhängigkeit der erfaßten physiologischen Parameter
- Empfangen zumindest eines Teils der Parameterdaten durch eine mobile Verarbeitungseinrichtung;
- Speichern zumindest eines Teils der der Verarbeitungseinrichtung übermittelten Parameterdaten in einer ortsfernen Auswerteeinrichtung;
- in Signalverbindung bringen zumindest einer Ausgabevorrichtung für Medikamente, vorzugsweise als halb- oder vollautomatischer Medikamentenspender ausgebildet, mit der Datenerfassungseinrichtung und/oder der Verarbeitungseinrichtung;
- Gegebenenfalls Eingeben von mit dem zumindest einen erfaßten physiologischen Parameter in Zusammenhang stehenden Zusatzinformationen; und
- Übermitteln von eigenständig durch die Ausgabevorrichtung für Medikamente erfassten bzw. erzeugten Zusatzinformationen und/oder eingegebenen Zusatzinformationen an die Datenerfassungseinrichtung und/oder Verarbeitungseinrichtung.
   Das Überwachungsverfahren kann weiterhin vorzugsweise den Schritt aufweisen
- Nach Vergleich von gemessenen Parametern und Zusatzinformationen bezüglich eingegebener Medikamenteneinnahme und/oder -dosierung
- Ausgeben eines Signals an der Auswerteeinrichtung (10) und/oder Verarbeitungseinrichtung (6) und/oder Datenerfassungseinrichtung (2) zur Anzeige von Informationen bezüglich einer veränderten oder bestätigten Medikamenteneinnahme und/oder -dosierung.

Die Merkmale und Vorteile des erfindungsgemäßen Überwachungssystems können ebenfalls in dem erfindungsgemäßen Überwachungsverfahren Anwendung finden.

Weiterhin erfindungsgemäß ist ein Computerprogrammprodukt vorgesehen, welches Programmteile zur Durchführung des erfindungsgemäßen Überwachungsverfahrens umfasst. Somit ist es Vorteilhafterweise möglich, das erfindungsgemäße Überwachungsverfahren auf einem Computer auszuführen.

Die Erfindung wird nachfolgend anhand begleitender Zeichnungen bevorzugter Ausführungsformen beispielhaft beschrieben. Es zeigen:
Fig. 1 ein schematisches Schaltungsdiagramm einer ersten Ausführungsform der vorliegenden Erfindung.
Fig. 2 ein schematisches Schaltungsdiagramm einer zweiten Ausführungsform der vorliegenden Erfindung.

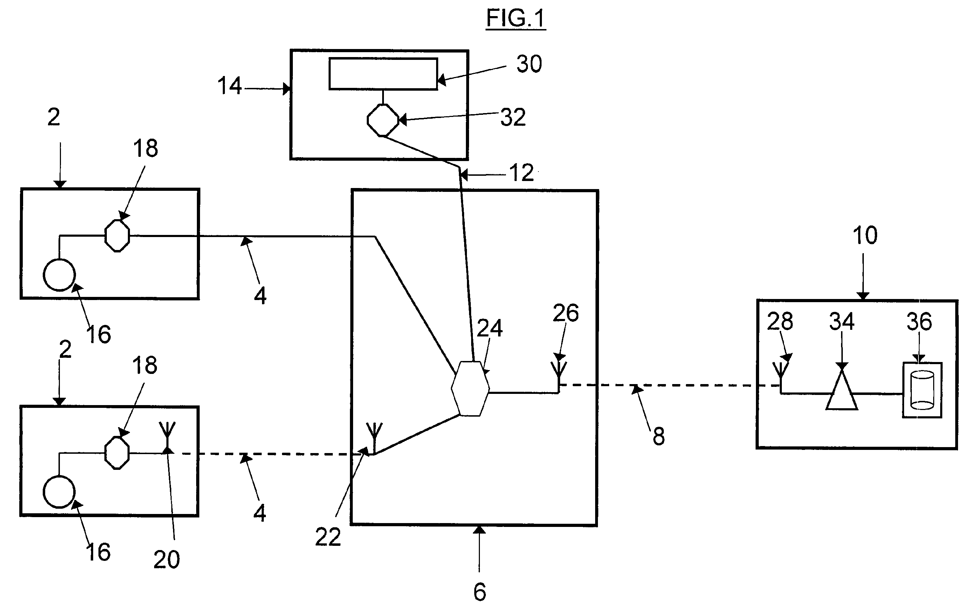

Das Überwachungssystem der in **Fig. 1** dargestellten ersten bevorzugten Ausführungsform der vorliegenden Erfindung weist zwei Datenerfassungseinrichtungen 2 auf. Die Datenerfassungseinrichtungen 2 dienen der Erfassung von physiologischen Parametern einer Person. Die Datenerfassungseinrichtung 2 steht über eine Signalverbindung 4 mit einer Verarbeitungseinrichtung 6 in Kontakt bzw. Signalverbindung bzw. Verbindung. Die Verarbeitungseinrichtung 6 ist vorgesehen, um insbesondere die von der Datenerfassungseinrichtung 2 übermittelten Parameterdaten zu verarbeiten und steht über eine Signalverbindung 8 mit einer Auswerteeinrichtung 10 in Kontakt bzw. Signalverbindung. Darüber hinaus steht eine Eingabeeinrichtung 14 mit der Verarbeitungseinrichtung 6 über eine Signalverbindung 12 in Verbindung.

Die Datenerfassungseinrichtung 2 ist ausgelegt, physiologische Parameter einer Person zu erfassen. Als physiologische Parameter im Sinne dieser Erfindung werden insbesondere Parameter, wie. z.B. Blutdruck, Herzschlag, Körpertemperatur, der Sauerstoffsättigungsgehalt des Bluts, Atmung (Frequenz und Volumen) , EKG sowie die Lage und Bewegung bzw. Beschleunigung des Körpers im Raum. Die physiologischen Parameter werden insbesondere durch einen Sensor 16 in der Datenerfassungseinrichtung 2 erfasst. Als Sensorelemente können hier allgemein übliche Sensoren verwendet werden. Besonders bevorzugterweise werden zur Erfassung der Bewegung bzw. Lage der Person Sensoren verwendet, welche Beschleunigungs- und Rotationsbewegungen bzw. -kräfte messen. Hierzu körnen insbesondere mikromechanische Low-g-Halbleiterbeschleunigungssensoren verwendet werden, welche eine Auflösung bzw. Genauigkeit von wenigen Milli-g bzw. Grad pro Sekunde aufweisen. Somit ist es Vorteilhafterweise möglich, die Position und den Bewegungszustand der Person exakt zu bestimmen. Die im Sensor 16 erfassten Daten werden in einem Prozessor 18 eingespeist, welcher entsprechende Parameterdaten erzeugt. Die Parameterdaten werden über die Signalverbindung 4 an die Verarbeitungseinrichtung 6 übermittelt. Dies kann einerseits über eine drahtgebundene Signalverbindung 4 erfolgen, jedoch bevorzugterweise über eine drahtlose Verbindung 4. Hierfür werden die Parameterdaten über ein Funkübertragungsmittel 20 an ein in der Verarbeitungseinichtung 6 vorgesehenes Funkübertragungsmittel 22 gesendet. Insbesondere kann hierbei die Bluetooth, Infrarot oder WLAN Technologie verwendet werden.

Die Verarbeitungseinrichtung 6 weist einen Prozessor 24 auf, welcher ausgelegt ist, die von der Datenerfassungseinrichtung 2 über die Signalverbindung 4 erhaltenen Parameterdaten zu verarbeiten bzw. weiter zu übermitteln. Die Weitervermittlung der Parameterdaten an die Auswerteeinrichtung 10 erfolgt vorzugsweise über in der Verarbeitungseinrichtung 6 vorgesehene Funkübertragungsmittel 26, welche über die Signalverbindung 8 mit in der Auswerteeinrichtung 10 vorgesehenen Funkübertragungsmitteln 28 in Verbindung stehen. Ebenfalls mit dem Prozessor 24 in Signalverbindung steht die Eingabeeinrichtung 14.

Die Eingabeeinrichtung 14 steht vorzugsweise über die Signalverbindung 12 mit der Verarbeitungseinrichtung 6 in Verbindung. Sie kann jedoch ebenfalls zusätzlich oder alternativ über eine (nicht dargestellte) Signalverbindung mit der Datenerfassungseinrichtung 2 in Verbindung stehen. In der dargestellten Ausführungsform ist die Eingabeeinrichtung 14 als manuelle Eingabeeinrichtung 14 ausgebildet, d.h. sie verfügt über eine Tastatur 30 oder einer hierzu ähnlichen Vorrichtung. Über die Tastatur 30 ist es möglich, mit den physiologischen Parametern in Verbindung stehende Zusatzinformationen einzugeben. Derartige Zusatzinformationen können beispielsweise der Gemütszustand oder das Befinden, Schmerzen, Schlafverhalten oder ähnliche Zustände der Person darstellen. Ebenfalls ist es möglich, dass Körpergewicht einzugeben oder den Zeitpunkt und die Zeitdauer einer Behandlung oder der Einnahme eines bestimmten Medikaments.
Die Eingabeeinrichtung 14 ist somit ausgelegt, Zusatzinformationen, welche mit den Parameterdaten bzw. den im Sensor 16 gewonnenen Daten in Zusammenhang stehen durch eine aktive Handlung der Person oder einer dritten Person zu erhalten. Die über die Tastatur 30 eingegebenen Daten können vorzugsweise über einen Prozessor 32 in der Eingabeeinrichtung 14 weiterverarbeitet werden und entsprechend über die Signalverbindung 12 der Verarbeitungseinrichtung 6 übermittelt werden.

Die über die Funkübertragungsmittel 28 übermittelten Daten können vorzugsweise in einem in der Auswerteeinrichtung 10 vorgesehenen Prozessor 34 weiterverarbeitet werden. Mit dem Prozessor 34 in Verbindung stehend weist die Auswerteeinrichtung 10 eine Speichereinrichtung bzw. Datenbank 36 auf, um die von der Verarbeitungseinrichtung 6 übermittelten Daten zumindest teilweise zu speichern. Die Daten können hierbei in gesichertem Zustand (d.h. insbesondere verschlüsselt) in der Speichereinrichtung 36 abgelegt werden. In einer bevorzugten Ausführungsform weist die Auswerteinrichtung 10 eine zusätzliche Signalverbindung zu einer externen Stelle (beispielsweise einem Arzt) auf, welche die in der Auswerteeinrichtung 10 abgelegten Daten -vorzugsweise verschlüsselt - abfragen kann. Besonders bevorzugterweise ist die dritte, externe Stelle ausgebildet, der Auswerteeinrichtung 10 Daten zu übermitteln, beispielsweise Daten, mittels welchen die Datenerfassungseinrichtung 2 oder Verarbeitungseinrichtung 6 gesteuert werden können oder diesen Informationen zugeführt werden können.

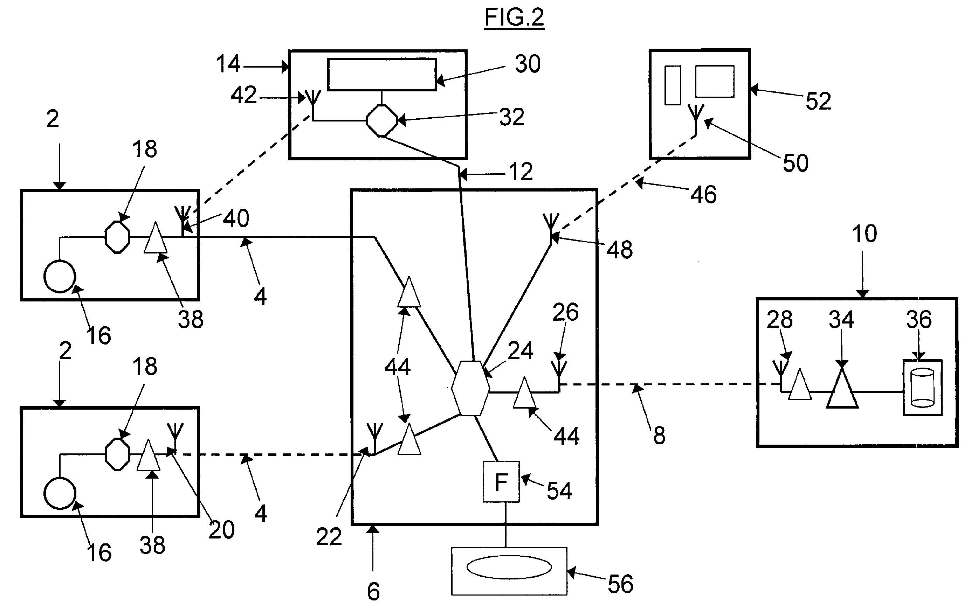

In **Fig. 2** ist ein schematisches Schaltungsdiagramm einer weiteren Ausführungsform der vorliegenden Erfindung dargestellt, wobei die mit der ersten Ausführungsform identischen Elemente mit gleichen Bezugsziffern versehen sind und nicht näher beschrieben werden. Die Datenerfassungseinrichtung 2 weist zusätzlich Datenerfassungs-Kodierungsmittel 38 auf. Durch die Kodierungsmittel 38 können die durch den Sensor 16 erfassten Daten gesichert werden. Die Sicherung kann hierbei vorzugsweise dreistufig erfolgen. In einem ersten Schritt werden die Rohdaten über ein Verschlüsselungsverfahren verschlüsselt. Die Verschlüsselung erfolgt beispielsweise über ein public-private-key-Verfahren. In einem zweiten Schritt werden die verschlüsselten Daten vorzugsweise mit einer Signatur versehen, wobei die Signatur beispielsweise über einen so genannten private-public-key-Verfahren erfolgt. Alternativ kann jedoch auch ein anderes Signaturverfahren verwendet werden, beispielsweise durch eine dem Sensor 16 bzw. der Datenerfassungseinrichtung 2 zugeordnete elektronische Seriennummer oder das Eichdatum bzw. die Eichziffer des Sensors 16. Um eine exakte und eindeutige Zuordenbarkeit des Systems zu der Person zu gewährleisten, können als Signaturmittel ebenfalls biometrische Merkmale angewandt werden, beispielsweise der Herzschlag, Fingerabdruck oder die Form der Iris, wobei hierbei insbesondere die über den Sensor 16 ermittelten physiologischen Parameter benutzt werden können. In einer dritten Stufe kann das so gesicherte Datenpaket vorzugsweise mit einer Check- bzw. Prüfsumme bzw. -ziffer versehen werden, um eine Manipulation der Daten erkennbar zu machen. Den Kodierungsmitteln 38 nachgeschaltet ist ein Funkübertragungsmittel 40, welches mit der Eingabeeinrichtung 14 bzw. darin angeordneten Funkübertragungsmitteln 42 in Verbindung steht. Die Eingabeeinrichtung 14 ist somit direkt mit wenigstens einer der Datenerfassungseinrichtungen 2 verbunden. Die Eingabeeinrichtung 14 kann nur mit wenigstens einer Datenerfassungseinrichtung 2 in Signalverbindung stehen, oder alternativ oder zusätzlich mit der Verarbeitungseinrichtung 6, wie in Fig. 1 dargestellt.

Die Verarbeitungseinrichtung 6 weist Verarbeitungseinrichtungs-Kodierungsmittel 44 auf, um die von der Datenerfassungseinrichtung 2 übermittelten Daten zu dekodieren oder weiter zu kodieren, bevor sie an die Auswerteeinrichtung 10 übermittelt werden. Über eine Signalverbindung 46, welche vorzugsweise über Funkübertragungsmittel 48, 50 drahtlos erfolgt, steht die Verarbeitungseinrichtung 6 mit einer Ausgabevorrichtung für Medikamente bzw. einem Medikamentenspender 52 in Verbindung. In dieser Ausführungsform erfüllt der Medikamentenspender 52 somit die Funktion einer zweiten Eingabeeinrichtung. Somit ist der Medikamentenspender 52 ausgelegt, Zusatzinformation, welche im Zusammenhang mit den physiologischen Parametern stehen, an die Verarbeitungseinrichtung 6 zu senden. Dies kann beispielsweise dadurch erfolgen, dass der Medikamentenspender 52 nach Ausgabe eines Medikaments automatisch eine Information an die Verarbeitungseinrichtung 6 übermittelt oder vor Ausgabe des Medikaments der Benutzer aufgefordert wird, eine Information einzugeben, welche an die Verarbeitungseinrichtung 6 übermittelt wird. Selbstverständlich kann der Medikamentenspender 52 auch mit zumindest einer der Datenerfassungseinrichtungen 2 in Verbindung stehen.

Die Datenerfassungseinrichtung weist darüber hinaus eine Feedbackeinrichtung 54 auf, welche eine Ausgabeeinrichtung 56 zum Vermitteln von Feedbackinformationen an die Person umfasst. Die Feedbackeinrichtung 54 umfasst Erinnerungsmittel, welche ausgelegt sind, über die Ausgabeeinrichtung 56 ein Erinnerungssignal auszugeben, welches die Person auffordert, eine vorbestimmbare Aktion auszuführen. Dies kann beispielsweise die Einnahme eines Medikaments sein oder eine vorbestimmte gymnastische Übung. Das Erinnerungssignal kann in Abhängigkeit eines vorbestimmten bzw. vorbestimmbaren Algorithmus ausgegeben werden, beispielsweise durch einen Algorithmus, welcher das Erinnerungssignal in zeitlicher Abhängigkeit ausgibt (beispielsweise zu einer bestimmten Tageszeit). Zusätzlich oder alternativ kann das Erinnerungssignal auch in Abhängigkeit von den Parameterdaten, den Zusatzinformationen oder den von der Auswerteeinrichtung 10 an die Verarbeitungseinrichtung 6 oder Datenerfassungseinrichtung 2 zurück übermittelten Auswertedaten ausgegeben werden. So kann als Erinnerungssignal beispielsweise die Aufforderung erfolgen, ein Medikament einzunehmen, wenn den Parameterdaten oder Zusatzinformationen zu entnehmen ist, dass sich die Person in einem schwachen körperlichen Zustand befindet.

Es wird somit ein Überwachungssystem geschaffen, welches in verschiedensten Anwendungsgebieten Verwendung findet. So kann es z.B. bei klinischen Tests, Langzeituntersuchungen, Verbesserung der Compliance von Patienten, Teleüberwachung, Telewerbung, Krankheitsmanagement, Rehabilitation, Gewichtsmanagement und Aktivitätsmessungen verwendet werden. Dies erfolgt, indem ein System geschaffen werden kann, welches einerseits Daten von der Person erfasst (aktiv und passiv) und entsprechend Feedback an diese zurückgibt. Die Erfassung der Daten erfolgt einerseits über die Sensoren 16 und andererseits unter aktiver Mithilfe der Person oder einer dritten Person über die Eingabeeinrichtung 14. Insbesondere können hierbei Daten eingegeben werden, welche sich auf verschiedenste physiologische Parameter beziehen. Hierzu zählen insbesondere Blutdruck, Puls, Atmung, Glukosespiegel, Gewicht, körperlicher Zustand, Schmerzzustand und das Schlafverhalten. Als Feedback an die Person können als Auswertedaten insbesonders physiologische Parameter bzw. deren Ergebnisse bzw. Verläufe(Trends) übermittelt werden, wie auch entsprechende Empfehlungen oder Erinnerungen, ein Medikament einzunehmen oder eine Messung vorzunehmen. Ferner ist es möglich, an der Ausgabeeinrichtung 56 Motivationsinformationen auszugeben, welche bewirken, dass eine Person eine bestimmte Aktion durchführt. Darüber hinaus wäre es denkbar, die Auswerteeinrichtung ebenfalls für Werbezwecke zu nutzen.

Es wird folglich ein Überwachungssystem bereitgestellt, welches primär eine fehlerfreie bzw. zuverlässige Erfassung bzw. Protokollierung von physiologischen Parametern über einen gewissen Zeitraum ermöglicht. Darüber hinaus wird durch das Überwachungssystem in einer bevorzugten Ausführungsform ermöglicht, an die Person ein Feedback in Abhängigkeit von den ermittelten physiologischen Parametern zu übermitteln.

### Bezugszeichenliste

- 2: Datenerfassungseinrichtung
- 4: Signalverbindung
- 6: Verarbeitungseinrichtung
- 8: Signalverbindung
- 10: Auswerteeinrichtung
- 12: Signalverbindung
- 14: Eingabeeinrichtung
- 16: Sensor
- 18: Prozessor
- 20: Funkübertragungsmittel
- 22: Funkübertragungsmittel
- 24: Prozessor
- 26: Funkübertragungsmittel
- 28: Funkübertragungsmittel
- 30: Tastatur
- 32: Prozessor
- 34: Prozessor
- 36: Speichereinrichtung
- 38: Kodierungsmittel
- 40: Funkübertragungsmittel
- 42: Funkübertragungsmittel
- 44: Kodierungsmittel
- 46: Signalverbindung
- 48: Funkübertragungsmittel
- 50: Funkübertragungsmittel
- 52: Medikamentenspender
- 54: Feedbackeinrichtung
- 56: Ausgabeeinrichtung

## Patentansprüche

1. Überwachungssystem zur Erfassung von physiologischen Parametern einer Person, umfassend
- zumindest eine an der Person angeordnete Datenerfassungseinrichtung (2) , welche ausgelegt ist, einen physiologischen Parameter zu erfassen und entsprechende Parameterdaten zu erzeugen;
- zumindest eine mobile Verarbeitungseinrichtung (6) , welche mit der Datenerfassungseinrichtung (2) in Signalverbindung steht und zum Empfang zumindest eines Teils der Parameterdaten ausgelegt ist;
- zumindest eine Eingabeeinrichtung (14) , welche mit der Verarbeitungseinrichtung (6) und/oder der Datenerfassungseinrichtung (2) in Signalverbindung steht und über welche mit dem zumindest einen erfassten physiologischen Parameter in Zusammenhang stehende Zusatzinformationen eingebbar sind; und
- zumindest eine ortsferne Auswerteeinrichtung (10) zum Speichern zumindest eines Teils der der Verarbeitungseinrichtung (6) übermittelten Daten.

2. Überwachungssystem nach Anspruch 1, wobei die Verarbeitungseinrichtung (6) und/oder die Datenerfassungseinrichtung (2) eine Feedbackeinrichtung (54) aufweisen, welche eine Ausgabeeinrichtung (56) zum Vermitteln von Feedbackinformationen an die Person umfasst.

3. Überwachungssystem nach Anspruch 1 oder 2, wobei die Verarbeitungseinrichtung (6) und/oder die Datenerfassungseinrichtung (2) ausgebildet sind, Auswertedaten von der Auswerteeinrichtung (10) zu erhalten.

4. Überwachungssystem nach einem der Ansprüche 2 oder 3, wobei die Feedbackeinrichtung (54) Erinnerungsmittel umfasst, welche ausgelegt sind, über die Ausgabeeinrichtung (56) ein Erinnerungssignal zur Aufforderung der Person, eine vorzugsweise vorbestimmbare Aktion durchzuführen, auszugeben.

5. Überwachungssystem nach Anspruch 4, wobei die Erinnerungsmittel ausgebildet sind, das Erinnerungssignal nach einem vorbestimmten bzw. vorbestimmbaren Algorithmus und/oder in Abhängigkeit von den Parameterdaten, den Zusatzinformationen und/oder den Auswertedaten auszugeben.

6. Überwachungssystem nach einem der vorhergehenden Ansprüche, wobei die Eingabeeinrichtung (14) als Ausgabevorrichtung für Medikamente, vorzugsweise als halb- oder vollautomatischer Medikamentenspender (52) ausgebildet ist, welche ausgelegt ist, zumindest einen Teil der Zusatzinformationen an die Datenerfassungseinrichtung (2) und/oder Verarbeitungseinrichtung (6) zu übermitteln.

7. Überwachungssystem nach einem der Ansprüche 2 bis 6, wobei die Ausgabeeinrichtung (56) ausgebildet ist, als Feedbackinformation zumindest einen Teil der Parameterdaten, zumindest einen Teil der Zusatzinformationen und/oder zumindest einen Teil der Auswertedaten auszugeben.

8. Überwachungssystem nach einem der vorhergehenden Ansprüche, wobei die Verarbeitungseinrichtung eine Eingabeeinrichtung aufweist.

9. Überwachungssystem nach einem der vorhergehenden Ansprüche, wobei die Signalverbindung zumindest zwischen Verarbeitungseinrichtung (6) und Auswerteeinrichtung (10) als gesicherte Verbindung ausgebildet ist, welche vorzugsweise über in der Verarbeitungseinrichtung (6), der Auswerteeinrichtung (10) und/oder der Datenerfassungseinrichtung (2) vorzugsweise modular angeordnete Kodierungsmittel (38, 44) bereitgestellt wird.

10. Überwachungssystem nach einem der vorhergehenden Ansprüche, wobei die Eingabeeinrichtung Mittel zur Authentifizierung aufweist.

11. Überwachungsverfahren zur Erfassung von physiologischen Parametern einer Person, umfassend die Schritte:
- Erfassen von physiologischen Parametern einer Person durch zumindest eine an dieser angeordneten Datenerfassungseinrichtung (2);
- Erzeugen entsprechender Parameterdaten in Abhängigkeit der erfaßten physiologischen Parameter;
- Empfangen zumindest eines Teils der Parameterdaten durch eine mobile Verarbeitungseinrichtung (6)
- Speichern zumindest eines Teils der der Verarbeitungseinrichtung (6) übermittelten Parameterdaten in einer ortsfernen Auswerteeinrichtung (10)
- in Signalverbindung bringen zumindest Eingabeeinrichtung (14) mit der Datenerfassungseinrichtung (2) und/oder der Verarbeitungseinrichtung (6); und
- Eingeben von mit dem zumindest einen erfaßten physiologischen Parameter in Zusammenhang stehenden Zusatzinformationen über die Eingabeeinrichtung (14).

12. Überwachungsverfahren nach Anspruch 11, weiterhin umfassend den Schritt:
Ausgeben eines Erinnerungsignals durch Erinnerungsmittel einer Feedbackeinrichtung (54) der Verarbeitungseinrichtung (6) und/oder der Datenerfassungseinrichtung (2) über eine Ausgabeeinrichtung (56)

13. Computerprogrammprodukt, welches Programmteile zur Durchführung eines Verfahrens nach einem der Ansprüche 11 oder 12 umfasst.
